# EUROPÄISCHE PATENTSCHRIFT

(11) **EP 2 533 823 B1**
(45) Veröffentlichungstag und Bekanntmachung des Hinweises auf die Patenterteilung: **09.01.2019**
(21) Anmeldenummer: 11704017.0
(22) Anmeldetag: 04.02.2011
(51) Int. Cl.: A61M 1/00, A61M 1/06, A61M 39/10

(54) **VORRICHTUNG UND VERFAHREN ZUR ERKENNUNG VON KOPPLUNGEN ZWISCHEN ZWEI SYSTEMKOMPONENTEN**
DEVICE AND METHOD FOR IDENTIFYING COUPLINGS BETWEEN TWO SYSTEM COMPONENTS
DISPOSITIF ET PROCÉDÉ DE DÉTECTION DE COUPLAGES ENTRE DEUX COMPOSANTS D'UN SYSTÈME

(30) Priorität: 11.02.2010 CH 1662010
(43) Veröffentlichungstag der Anmeldung: 19.12.2012
(73) Patentinhaber: Medela Holding AG, 6340 Baar (CH)
(72) Erfinder: VISCHER, Peter, CH-6403 Küssnacht am Rigi (CH); WÄCKERLIN, Daniela, CH-6340 Baar (CH); KOCH, Urs, CH-6404 Greppen (CH)
(74) Vertreter: Clerc, Natalia
(86) Internationale Anmeldenummer: PCT/CH2011/000019
(87) Internationale Veröffentlichungsnummer: WO 2011/097744

(56) Entgegenhaltungen:
- WO-A1-2009/083943
- WO-A1-2009/144726
- DE-A1- 10 005 108

## Beschreibung

### TECHNISCHES GEBIET

Die vorliegende Erfindung betrifft eine Vorrichtung und ein Verfahren zur Erkennung von Kopplungen zwischen zwei Komponenten eines Systems gemäss Oberbegriff des Patentanspruchs 1 bzw. 14.

### STAND DER TECHNIK

Üblicherweise wird eine Kopplung zwischen zwei Systemkomponenten immer dann ermöglicht, wenn sich zwei Kopplungsteile, welche die Verbindung zwischen den zwei Systemkomponenten erstellen sollen, einander physisch ergänzen und zwei Teile einer Einheit bilden. Dies ist anhand einer Steckverbindung einfach zu erkennen.

Insbesondere im medizinaltechnischen Bereich reicht jedoch dieses Kriterium oft nicht aus. So offenbart beispielsweise US 2006/0073048 eine Irrigationspumpe mit einem auswechselbaren Schlauchset und einer Steuerkonsole. Das Schlauchset beinhaltet eine Radiofrequenz-Identifikations-Vorrichtung (RFID), in welcher physische Merkmale des Schlauchsets gespeichert sind. Eine Steuerungskonsole liest diese Daten und entscheidet unter Berücksichtigung von weiteren Daten, ob die Pumpe gemeinsam mit diesem Schlauchset geeignet ist, Flüssigkeit in der gewünschten Menge zu fördern. Des Weiteren kann die Steuerungskonsole anhand einer Herstelleridentifikation auch prüfen, ob das Schlauchset vom Pumpenhersteller überhaupt zur Verwendung mit seiner Pumpe zugelassen ist.

US 5 460 490 beschreibt ebenfalls eine Irrigations- und Saugpumpe mit mehreren Schlauchsets. Die verschiedenen Schlauchsets sind jeweils nur für eine spezifische Anwendung zugelassen und entsprechend codiert. Die Pumpe verfügt über entsprechende Erkennungsmittel, um diese Codes zu lesen.

US 7 237 990 zeigt ein chirurgisches Schneidwerkzeug mit einer RFID im Handgriff, um Daten zwischen Messer und Handgriff auszutauschen.

WO 03/013372 offenbart ein chirurgisches System mit einem Handgriff und mit Zubehör, welches an den Handgriff befestigt werden kann. Auch hier ist zur Erkennung des Zubehörs ein Chip im Zubehör angeordnet.

WO 2009/144726 beschreibt zwei Bauteile eines medizinischen Geräts mit einer RF Erkennungseinheit, welche mit einem Prozessor in einem Bauteil verbunden ist, wobei der Prozessor in Abhängigkeit des erhaltenen Signals eine Energiequelle im anderen Bauteil aktiviert oder nicht.

Diese bekannten Systeme sind erfolgreich, um falsche Bedienungseinstellungen der Geräte zu vermeiden, da das System selbst die sich aus der Kombination von Gerät und Zubehör ergebende Funktionsweise wählt oder zumindest vorschlägt.

Wird jedoch versucht, ein nicht passendes Zubehör an das Gerät anzuschliessen, so kann dies zu physischen Schäden am Gerät, insbesondere im Bereich seines Kopplungselements, führen. Des Weiteren ist nicht immer sichergestellt, dass das Gerät nicht trotzdem benützt werden kann.

### DARSTELLUNG DER ERFINDUNG

Es ist deshalb eine Aufgabe der Erfindung, eine Vorrichtung und ein Verfahren zur Erkennung von Kopplungen zwischen zwei Komponenten eines Systems zu schaffen, welche eine gemeinsame Benützung der Systemkomponenten nur bei einer zulässigen Kopplung erlauben.

Diese Aufgabe lösen eine Vorrichtung und ein Verfahren mit den Merkmalen der Patentansprüche 1 bzw. 14.

In der erfindungsgemässen Vorrichtung zur Erkennung von Kopplungen zwischen einer ersten und einer zweiten Komponente eines Systems ist eine der zwei Komponenten mit einer Identifikationseinheit versehen. Eine Prüfeinheit steht mit der zweiten Komponente in Wirkverbindung. Die Identifikationseinheit enthält eine Information zur ersten Komponente. Die Prüfeinheit ist zur Erkennung und Verarbeitung dieser Information ausgebildet. Die Prüfeinheit ist ferner so ausgebildet, dass sie in Abhängigkeit eines Ergebnisses der Verarbeitung der Information eine Kopplung zwischen der ersten und der zweiten Komponente zulässt oder verhindert.

Im erfindungsgemässen Verfahren zur Erkennung von Kopplungen zwischen einer ersten und einer zweiten Komponente eines Systems enthält eine der zwei Komponenten eine Information zu dieser ersten Komponente, wobei eine Prüfeinheit diese Information erkennt und verarbeitet und eine Wirkung auf die zweite Komponente ausübt. Die Prüfeinheit lässt in Abhängigkeit eines Ergebnisses der Verarbeitung der Information eine Kopplung zwischen der ersten und der zweiten Komponente zu oder verhindert.

Dadurch wird sichergestellt, dass nur gewünschte Verbindungen zwischen den zwei Systemkomponenten, insbesondere einer Saugpumpe und einem Zubehör, erstellt werden können bzw. dass die Systemkomponenten nur bei zulässiger Kombination überhaupt miteinander in Einsatz gelangen können. Dadurch können markenfremde, ungeeignete, veraltete oder anders artige nicht geeignete Systemkomponenten erkannt und ihr Einsatz bzw. ihre Kopplung vermieden werden.

Zusätzlich oder alternativ kann die Prüfeinheit in Abhängigkeit eines Ergebnisses der Verarbeitung der Informationen einen Betrieb der zweiten Komponente, welche in diesem Fall eine Saugpumpe ist, zulassen oder verhindern.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: ein Prinzipschema der erfindungsgemässen Vorrichtung;
- Figur 2: eine schematische Darstellung der erfindungsgemässen Vorrichtung in einer ersten Ausführungsform;
- Figur 3: eine schematische Darstellung der erfindungsgemässen Vorrichtung in einer zweiten Ausführungsform und
- Figur 4: eine schematische Darstellung der erfindungsgemässen Vorrichtung in einer dritten Ausführungsform.

### BESCHREIBUNG BEVORZUGTER AUSFÜHRUNGSFORMEN

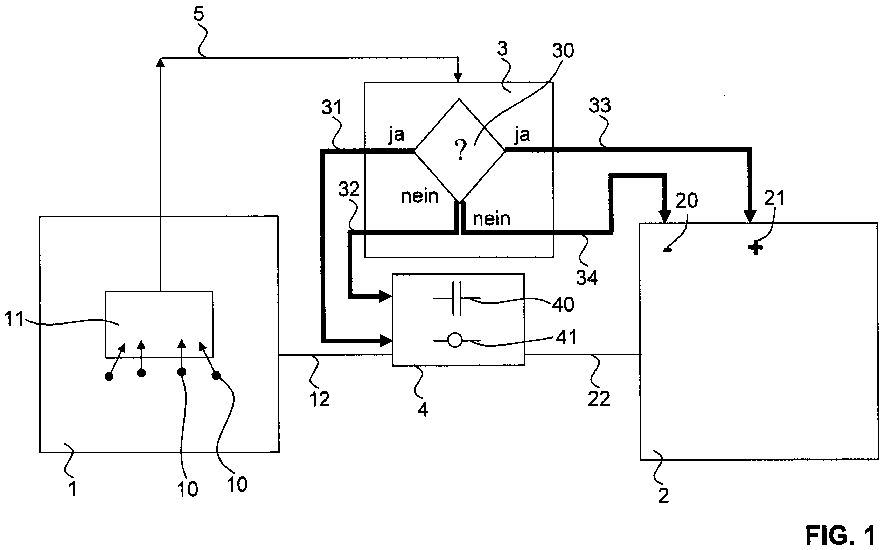

In Figur 1 ist ein Prinzipschema der erfindungsgemässen Vorrichtung dargestellt, welches auch das erfindungsgemässe Verfahren erläutert.

Eine erste Systemkomponente 1 verfügt über eine Identifikationseinheit 11, in welcher mindestens eine Information, vorzugsweise mehrere Informationen zu Eigenschaften 10 dieser ersten Systemkomponente 1 gespeichert sind.

Eine zweite Systemkomponente 2 ist über eine Kopplungseinheit 4 mit der ersten Systemkomponente 1 verbindbar. Die Kopplung erfolgt vorzugsweise durch Verbindung eines ersten und eines zweiten Kopplungsteils 12, 22 miteinander.

Eine Prüfeinheit 3, auch Controller genannt, weist eine zentrale Intelligenz oder Logik 30 auf und steht in Wirkverbindung mit der ersten und der zweiten Systemkomponente 1, 2. Soll nun eine Kopplung zwischen der ersten und der zweiten Systemkomponente 1, 2 stattfinden, so werden die in der Identifikationseinheit 11 gespeicherten Informationen bzw. Daten an die Prüfeinheit 3 übermittelt. Dies ist in der Figur 1 mit der Bezugsziffer 5 gekennzeichnet. Die Übermittlung kann aktiv und selbsttätig von der ersten Systemkomponente 1 erfolgen oder sie kann von der Prüfeinheit 3 aktiviert werden.

In der Prüfeinheit 3 sind Informationen zur zweiten Systemkomponente 2 gespeichert. Alternativ können diese Informationen ebenfalls abgefragt oder an die Prüfeinheit 3 übermittelt werden. Die Prüfeinheit 3, insbesondere die Logik 30, prüft nun, ob die erste Systemkomponente 1 geeignet ist, um mit der zweiten Systemkomponente 2 eine Verbindung einzugehen. Vorzugsweise wird über einen geeigneten Algorithmus eine Steuergrösse bestimmt.

Ist die Antwort ,ja", so kann nun eine Kopplung, d.h. eine Verbindung des ersten und zweiten Kopplungsteils 12, 22, zugelassen werden. Das entsprechende Signal von der Prüfeinheit 3 an die Kopplungseinheit 4 ist in der Figur 1 mit der Bezugsziffer 31 versehen. Die erstellte Kopplung weist die Bezugsziffer 41 auf.

Ist die Antwort "nein", so wird eine Kopplung verhindert. Das heisst, das erste und das zweite Kopplungsteil 1, 2 können nicht miteinander verbunden werden. Das entsprechende Signal von der Prüfeinheit 3 an die Kopplungseinheit 4 ist in der Figur 1 mit der Bezugsziffer 32 versehen. Die verhinderte Kopplung weist die Bezugsziffer 40 auf. Die Verhinderung der Kopplung kann beispielsweise dadurch erfolgen, dass ein Steckereingang nicht freigegeben wird, indem sich insbesondere eine Schutzplatte vor einem Kopplungselement nicht entfernen lässt.

In Figur 1 ist ferner eine weitere Aktion der Prüfeinheit 3 dargestellt. Ist die oben genannte Antwort ,ja", so wird ein Signal an die zweite Systemkomponente 2 übermittelt (siehe Bezugsziffer 33) und ein Betrieb der zweiten Systemkomponente 2 wird zugelassen (siehe Bezugsziffer 21). Ist die oben genannte Antwort "nein", so wird ebenfalls ein Signal an die zweite Systemkomponente 2 übermittelt (siehe Bezugsziffer 34) und der Betrieb der zweiten Systemkomponente 2 wird verhindert (siehe Bezugsziffer 20).

Es ist auch möglich, dass bei Antwort "nein" kein Signal übermittelt wird und die Kopplung bzw. der Betrieb der zweiten Systemkomponente 2 einfach nicht ermöglicht wird.

Vorzugsweise ist die Prüfeinheit 3 in die zweite Systemkomponente 2 integriert oder zumindest in einem gemeinsamen Gehäuse angeordnet. Sie kann jedoch auch als getrenntes Bauteil mit eigenem Gehäuse ausgebildet sein.

In einer bevorzugten Ausführungsform der erfindungsgemässen Vorrichtung und in einer bevorzugten Variante des erfindungsgemässen Verfahrens werden beide Aktionen getätigt: d.h. es werden sowohl die Kopplung als auch der Betrieb der zweiten Systemkomponente 2 zugelassen oder verhindert. In anderen Ausführungsformen ist die Vorrichtung so ausgebildet, dass nur eine der zwei Aktionen vorgesehen ist. D.h. in einer ersten Ausführungsform kann die Vorrichtung die Kopplung ermöglichen oder verhindern. In einer zweiten Ausführungsform kann die Vorrichtung den Betrieb der zweiten Systemkomponente ermöglichen oder verhindern.

Im Falle, dass nur der Betrieb ermöglicht oder verhindert werden kann, weist die zweite Systemkomponente eine Saugpumpe auf, wobei deren Betrieb ermöglicht oder verhindert wird. Die erste Systemkomponente 1 ist in diesem Fall vorzugsweise ein Saugschlauch, beispielsweise ein Drainageschlauch oder ein Vakuumschlauch.

In allen Beispielen ist die zweite Systemkomponente 2 vorzugsweise eine Saugpumpe, beispielsweise eine Drainagepumpe zum Absaugen von Körperflüssigkeiten oder eine Brustpumpe zum Abpumpen menschlicher Muttermilch. Derartige Drainagepumpen kommen beispielsweise bei der Wunddrainage, bei der Thoraxdrainage, bei Operationen und bei Fettabsaugungen zum Einsatz.

In allen Beispielen ist die erste Systemkomponente 1 vorzugsweise ein Zubehör zu dieser Saugpumpe. Beispielsweise ist sie eine Brusthaube für eine Brustpumpe, ein Vakuumschlauch zur Verbindung zwischen Brusthaube und Brustpumpe, eine vakuumbeaufschlagte Wundeinlage, ein Drainageschlauch, ein Sekretschlauch, ein Serviceschlauch, ein Katheter oder ein Fluidsammelbehälter.

In allen Ausführungsformen sind das erste Kopplungselement 12 und das zweite Kopplungselement 22 zwei einander ergänzende Teile einer Verbindung, beispielsweise einer Steckverbindung, einer Schnappverbindung, einer Drehverbindung, einer Bajonettverbindung, einer Magnetverbindung oder einer elektromechanischen Verbindung.

Die Identifikationseinheit 11 ist vorzugsweise in allen Ausführungsformen Bestandteil der ersten Systemkomponente oder gemeinsam mit ihr in demselben Gehäuse angeordnet. Sie kann beispielsweise in der Brusthaube, im Fluidsammelbehälter, im oder am Schlauch, in oder an der Schlauchinnenwandung oder -aussenwandung, an der Schnittstelle zwischen Drainagebehälter und Drainagebeutel oder an der Schnittstelle zwischen Drainageschlauch und Wunde angeordnet sein.

Die Erkennung bzw. Identifikation erfolgt vorzugsweise nicht mechanisch, sondern elektronisch. Bevorzugt ist eine Radiofrequenzidentifikation (RFID) im Einsatz, wobei die entsprechende Vorrichtung in der Prüfeinheit 3 vorgesehen ist. Die Übermittlung, Erkennung und/oder Verarbeitung der Informationen kann jedoch auch elektronisch, optisch oder chemisch erfolgen. Vorzugsweise werden die Informationen jedoch berührungslos übertragen.

Die Informationen und Daten, welche in der Identifikationseinheit 11 gespeichert sind, sind vorzugsweise Informationen des Herstellers, wie Marke, Typenbezeichnung, Alter der Systemkomponente oder Herstellungsdatum.

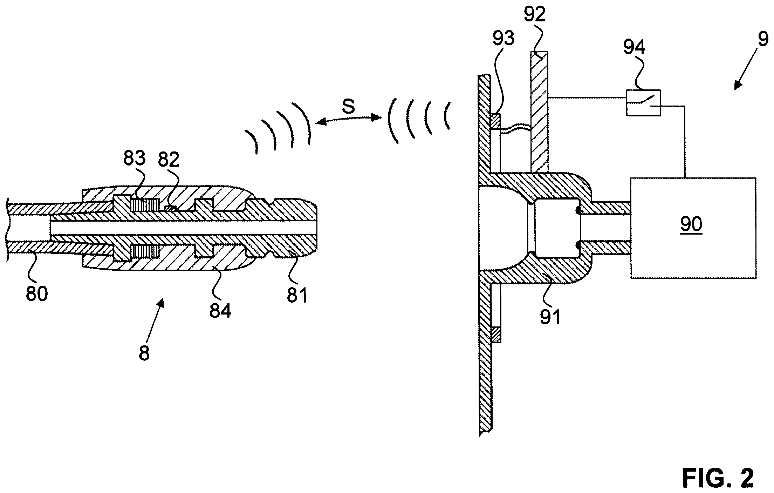

In Figur 2 ist eine erste Anwendung der erfindungsgemässen Vorrichtung dargestellt. Die erste Systemkomponente 8 ist ein Schlauch 80 mit einem ersten Steckerteil 81. Im Gehäuse 84 des Steckers 81 ist eine Identifikationseinheit angeordnet, hier ein Mikrochip 82 mit gespeicherten Informationen und eine Transponderspule 83 für RIFD.

Die zweite Systemkomponente 9 ist hier eine Saugpumpe 90, deren Gehäuse mit einem zweiten Steckerteil 91 zur Verbindung mit dem ersten Steckerteil 81 versehen ist. Das erste Steckerteil 81 ist vorzugsweise männlich, das zweite Steckerteil 91 weiblich ausgebildet. Eine Elektronik 92 ist mit einer RFID oder einer anderen berührungslosen Informationsabfrageeinheit versehen. Eine Antenne 93 übermittelt die von der ersten Systemkomponente 8 übermittelte Information an die Prüfeinheit, d.h. an die Elektronik 92. Je nach Art der erhaltenen Information wird nun von der Elektronik ein Schalter 94 geschlossen oder er bleibt geöffnet. Ist der Schalter 94 geöffnet, so ist der Betrieb der Saugpumpe verhindert. Wird er geschlossen, kann die Saugpumpe betrieben werden und der Schlauch 80 kann mit Unterdruck beaufschlagt werden. In diesem Beispiel wird somit je nach Antwort ,ja" oder "nein" der Betrieb der zweiten Systemkomponente 2 erlaubt oder verhindert.

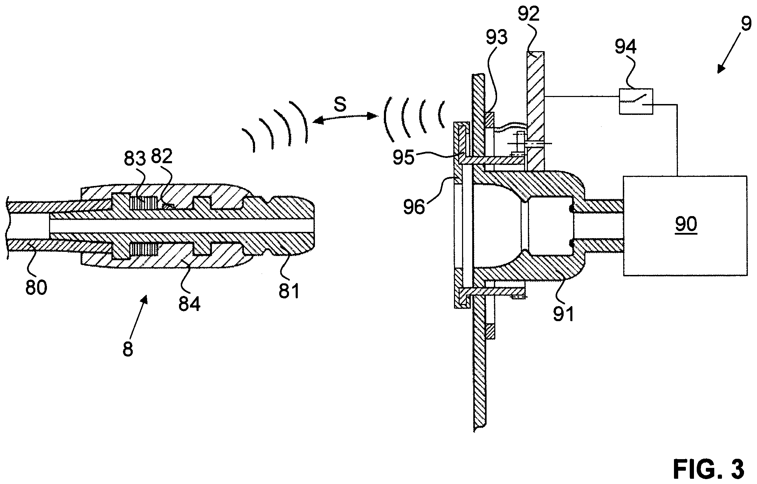

In Figur 3 ist eine weitere Ausführungsform dargestellt. Sie weist im wesentlichen dieselben Bauteile auf wie die Ausführungsform gemäss Figur 2. Gleiche Teile sind deshalb mit gleichen Bezugszeichen versehen und werden hier nicht mehr beschrieben. Zusätzlich ist hier jedoch eine Vorrichtung vorhanden, welche den Eingang des weiblichen Steckerteils 91 verschliesst. Diese Vorrichtung umfasst in diesem Beispiel ein Excenterrad 95 und eine damit wirkverbundene Verschlussplatte 96. Andere Lösungen sind möglich.

Vorzugsweise ist der Eingang des weiblichen Steckerteils 91 standardmässig mit der Verschlussplatte 96 verschlossen, so dass das männliche Steckerteil 81 nicht eingesteckt werden kann. Ist nun die Antwort auf die Abfrage "ja", so bewirkt die Elektronik 92 über einen hier nicht dargestellten Antrieb, dass das Excenterrad 95 die Verschlussplatte 96 bewegt und der Eingang des weiblichen Steckerteils freigegeben wird. Andere Varianten sind auch möglich. Dadurch kann die Kopplung zwischen erster und zweiter Systemkomponente stattfinden. Da die Antwort "ja" zusätzlich den Schalter 94 geschlossen hat, kann auch die Saugpumpe in Betrieb genommen werden.

Alternativ kann im Ausführungsbeispiel gemäss Figur 3 der Schalter 94 nicht vorhanden sein und lediglich der Eingang zum weiblichen Steckerteil 91 kann verschlossen sein.

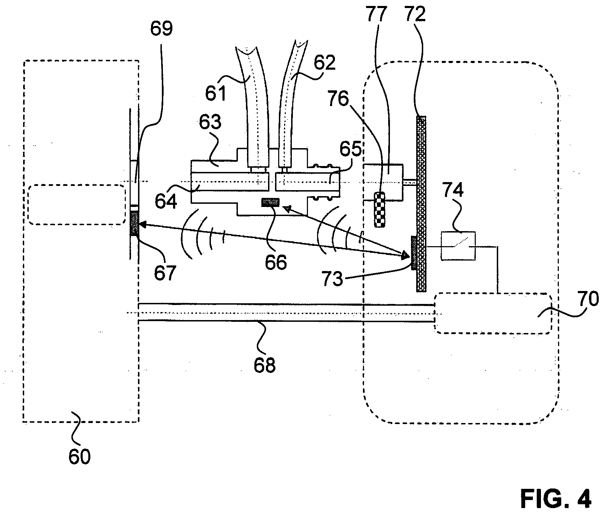

In Figur 4 ist ein weiteres Ausführungsbeispiel, nun aus dem Bereich der Drainagevorrichtungen, dargestellt. Die erste Systemkomponente ist ein Drainagebehälter 60, welcher mit einem Mikrochip 67 versehen ist. Auf diesem Mikrochip 67 sind Daten und Informationen zum Drainagebehälter gespeichert. Der Drainagebehälter 60 ist mit einem patientenseitigen Anschluss 69 versehen.

Die zweite Systemkomponente ist eine Saugpumpe 70, welche über eine Vakuumleitung bzw. einen Vakuumschlauch 68 mit dem Drainagebehälter 60 verbunden ist. Im Gehäuse der Saugpumpe 70 ist eine Prüfeinheit oder Elektronik 72 angeordnet. Auch hier ist wiederum ein Schalter 74 vorhanden, welcher mit der Elektronik 72 verbunden ist und geschlossen sein muss, um die Saugpumpe zu betreiben. Die Elektronik 72 ist mit einer RFID 73 versehen. Das Gehäuse ist mit einem patientenseitigen Anschluss 77 versehen, welcher vorzugsweise über einen mechanischen Schalter 76 verschliessbar ist. Der Schalter 76 wird ebenfalls mittels der Elektronik 72 betätigt.

Ein Doppelstecker 63 bildet die Kopplungseinheit und verbindet den Drainagebehälter 60 mit der Saugpumpe 70. Er weist einen behälterseitigen Anschluss 64 auf, welcher in den patientenseitigen Anschluss 69 des Drainagebehälters 60 einsteckbar ist und einen pumpenseitigen Anschluss 65, welcher in den patientenseitigen Anschluss 77 der Saugpumpe 70 einsteckbar ist. Im Doppelstecker 63 sind ferner ein Drainageschlauch 61 und vorzugsweise ein Serviceschlauch 62 eingesteckt. Im Gehäuse des Doppelsteckers 63 ist ein Mikrochip 66 angeordnet, in welchem Informationen und Daten zum Doppelstecker und vorzugsweise zu den Schläuchen 61, 62 gespeichert sind. Der Doppelstecker 63 bildet somit nicht nur eine Kopplungseinheit sondern zugleich auch eine weitere zweite Systemkomponente.

Die Daten aus den zwei Mikrochips 66, 67 der zwei zweiten Systemkomponenten 60, 63 werden an die Elektronik 72 der ersten Systemkomponente 70 gesandt, dort verarbeitet und aufgrund der erhaltenen Informationen wird der Schalter 74 geöffnet oder nicht. Ebenfalls aufgrund der erhaltenen Informationen wird der mechanische Schalter 76, d.h. die Barriere, geöffnet oder nicht. In diesem Beispiel müssen vorzugsweise beide zweiten Systemkomponenten die Erfordernisse erfüllen, damit die Kopplung zur ersten Systemkomponente erfolgen kann und die Saugpumpe in Betrieb genommen werden kann. Auch hier erfolgt die Datenübermittlung vorzugsweise berührungslos.

Die erfindungsgemässe Vorrichtung ermöglicht eine gemeinsame Benutzung von Systemkomponenten nur bei einer zulässigen Kopplung derselben.

**BEZUGSZEICHENLISTE**

| | | | |
|---|---|---|---|
| 1 | erste Systemkomponente | 65 | pumpenseitiger Anschluss |
| 10 | Eigenschaften der ersten Systemkomponente | 66 | Mikrochip |
| | | 67 | Mikrochip |
| 11 | Identifikationseinheit | 68 | Vakuumschlauch |
| 12 | erstes Kopplungselement | 69 | patientenseitiger Anschluss |
| | | | |
| 2 | zweite Systemkomponente | 70 | Saugpumpe |
| 20 | Aus | 72 | Elektronik |
| 21 | Ein | 73 | RFID |
| 22 | zweites Kopplungselement | 74 | Schalter |
| | | 76 | mechanischer Schalter |
| 3 | Prüfeinheit | 77 | patientenseitiger Anschluss |
| 30 | Logik | | |
| 31 | Ja | 8 | erste Systemkomponente |
| 32 | Nein | 80 | Drainageschlauch |
| 33 | Ja | 81 | erstes Steckerteil |
| 34 | Nein | 82 | Mikrochip |
| | | 83 | Transponderspule |
| 4 | Kopplungseinheit | 84 | Steckergehäuse |
| 40 | Kopplung verhindert | | |
| 41 | Kopplung ermöglicht | 9 | zweite Systemkomponente |
| | | 90 | Saugpumpe |
| 5 | Informationsfluss | 91 | zweites Steckerteil |
| | | 92 | Elektronik |
| 60 | Fluidsammelbehälter | 93 | Antenne |
| 61 | Drainageschlauch | 94 | Schalter |
| 62 | Serviceschlauch | 95 | Excenterrad |
| 63 | Doppelstecker | 96 | mechanischer Verschluss |
| 64 | behälterseitiger Anschluss | | |

## Patentansprüche

1. Vorrichtung zur Erkennung von Kopplungen zwischen einer ersten und einer zweiten Komponente eines Systems, wobei die Vorrichtung die zwei Komponenten, eine Identifikationseinheit und eine Prüfeinheit aufweist, wobei eine der zwei Komponenten mit der Identifikationseinheit versehen ist und wobei die Prüfeinheit mit der zweiten Komponente in Wirkverbindung steht, wobei die Identifikationseinheit eine Information zur ersten Komponente enthält und wobei die Prüfeinheit zur Erkennung und Verarbeitung dieser Information ausgebildet ist, wobei die Prüfeinheit ferner so ausgebildet ist, dass sie in Abhängigkeit eines Ergebnisses der Verarbeitung der Information eine Kopplung zwischen der ersten und der zweiten Komponente zulässt oder verhindert,
**dadurch gekennzeichnet, dass** die Prüfeinheit mit einer Barriere wirkverbunden ist und dass die Prüfeinheit mittels der Barriere eine Verbindung zwischen der ersten und der zweiten Komponente zulässt oder verhindert, wobei die Barriere bei einer unzulässigen Kopplung eine Verbindung zwischen dem ersten und dem zweiten Kopplungselement verhindert.

2. Vorrichtung nach Anspruch 1, wobei die zweite Komponente eine Brustpumpe zum Abpumpen menschlicher Muttermilch oder eine Drainagepumpe zum Abpumpen von Körperflüssigkeiten ist.

3. Vorrichtung nach einem der Ansprüche 1 oder 2, wobei die erste Komponente ein Zubehör der zweiten Komponente ist, vorzugsweise eine Brusthaube einer Brustpumpe zum Abpumpen menschlicher Muttermilch oder ein Saugschlauch oder ein Serviceschlauch oder eine Wundeinlage oder ein Fluidsammelbehälter oder ein Katheter.

4. Vorrichtung nach einem der Ansprüche 1 bis 3, wobei die erste Komponente ein erstes Kopplungselement und die zweite Komponente ein zweites Kopplungselement aufweist und wobei die zwei Kopplungselemente zur Erstellung der Kopplung miteinander verbunden werden.

5. Vorrichtung nach Anspruch 4, wobei das erste und das zweite Kopplungselement zwei einander ergänzende Teile einer Steckverbindung oder einer Schnappverbindung oder einer Drehverbindung oder einer Bajonettverbindung oder einer Magnetverbindung oder einer elektromechanischen Verbindung sind.

6. Vorrichtung nach Anspruch 4, wobei die Identifikationseinheit und die Prüfeinheit derart ausgebildet sind, dass eine Erkennung und Verarbeitung der Information bei nicht erfolgter Kopplung des ersten und zweiten Kopplungselements erfolgt.

7. Vorrichtung nach einem der Ansprüche 1 bis 6, wobei die Prüfeinheit eine Radiofrequenzidentifikationseinrichtung (RFID) aufweist.

8. Vorrichtung nach einem der Ansprüche 1 bis 7, wobei die Identifikationseinheit und die Prüfeinheit derart ausgebildet sind, dass die Übermittlung und/oder Erkennung und/oder Verarbeitung der Information elektronisch oder optisch oder chemisch erfolgt.

9. Vorrichtung nach einem der Ansprüche 1 bis 8, wobei die Identifikationseinheit und die Prüfeinheit derart ausgebildet sind, dass die Übermittlung der Information berührungslos erfolgt.

10. Vorrichtung nach einem der Ansprüche 1 bis 9, wobei die Erkennungseinheit mit einem Schalter wirkverbunden ist, welcher bei einer zulässigen Kopplung den Betrieb der Saugpumpe freigibt.

11. Vorrichtung nach Anspruch 1, wobei die Barriere am oder im zweiten Kopplungsteil angeordnet ist.

12. Vorrichtung nach einem der Ansprüche 1 bis 11, wobei das System ein Brustpumpensystem zum Absaugen von menschlicher Muttermilch oder ein Drainagesystem zum Absaugen von Körperflüssigkeiten ist.

13. Vorrichtung nach einem der Ansprüche 1 bis 12, wobei die Prüfeinheit ferner so ausgebildet ist, dass sie in Abhängigkeit des Ergebnisses der Verarbeitung der Information einen Betrieb der zweiten Komponente, welche in diesem Fall eine Saugpumpe ist, zulässt oder verhindert.

14. Verfahren zur Erkennung von Kopplungen zwischen einer ersten und einer zweiten Komponente eines Systems, wobei eine der zwei Komponenten eine Information zu dieser ersten Komponente enthält, wobei eine Prüfeinheit diese Information erkennt und verarbeitet und eine Wirkung auf die zweite Komponente ausübt, wobei die Prüfeinheit in Abhängigkeit eines Ergebnisses der Verarbeitung der Information eine Kopplung zwischen der ersten und der zweiten Komponente zulässt oder verhindert, **dadurch gekennzeichnet, dass** die Prüfeinheit bei einer zulässigen Kopplung eine Barriere öffnet, welche bei einer unzulässigen Kopplung eine Verbindung zwischen dem ersten und dem zweiten Kopplungselement verhindert.

## Claims

1. An apparatus for recognizing couplings between a first and a second component of a system, wherein the apparatus comprises the two components, an identification unit and a checking unit, wherein one of the two components is provided with the identification unit and wherein the checking unit is operatively connected to the second component, wherein the identification unit contains an item of information relating to the first component and wherein the checking unit is designed for recognizing and processing this item of information, wherein the checking unit is moreover designed such that, as a result of the processing of information, it allows a coupling between the first and the second component or it prevents it, **characterized in that** the checking unit is operatively connected to a barrier, wherein the checking unit allows or prevents the connection between the first and the second component by means of the barrier, wherein the barrier prevents a connection between the first and second coupling elements in the case of an inadmissible coupling.

2. The apparatus as claimed in claim 1, wherein the second component is a breastpump for expressing human breastmilk or a drainage pump for draining bodily fluids.

3. The apparatus as claimed in one of claims 1 and 2, wherein the first component is an accessory of the second component, preferably a breast shield of a breastpump for expressing human breastmilk or suction tubing or service tubing or a wound insert or a fluid collection container or a catheter.

4. The apparatus as claimed in one of claims 1 to 3, wherein the first component has a first coupling element and the second component has a second coupling element and wherein the two coupling elements are interconnected to establish the coupling.

5. The apparatus as claimed in claim 4, wherein the first and the second coupling elements are two complementary parts of a plug-in connection or a snap-on connection or a swivel connection or a bayonet connection or a magnetic connection or an electro-mechanical connection.

6. The apparatus as claimed in claim 4, wherein the identification unit and the checking unit are designed such that recognition and processing of the item of information is brought about when the coupling between the first and second coupling elements has not been brought about.

7. The apparatus as claimed in one of claims 1 to 6, wherein the checking unit has a radio-frequency identification (RFID) device.

8. The apparatus as claimed in one of claims 1 to 7, wherein the identification unit and the checking unit are designed such that the transmission and/or recognition and/or processing of the item of information is brought about electronically or optically or chemically.

9. The apparatus as claimed in one of claims 1 to 8, wherein the identification unit and the checking unit are designed such that the item of information is transmitted contactlessly.

10. The apparatus as claimed in one of claims 1 to 9, wherein the recognition unit is operatively connected to a switch that enables the operation of the suction pump in the case of an admissible coupling.

11. The apparatus as claimed in claim 1, wherein the barrier is arranged on or in the second coupling part.

12. The apparatus as claimed in one of claims 1 to 11, wherein the system is a breastpump system for expressing human breastmilk or a drainage system for draining bodily fluids.

13. The apparatus as claimed in one of claims 1 to 12, wherein as a function of a result of the processing of the item of the information, the checking unit allows or prevents operation of the second component, which in this case is a suction pump.

14. A method for recognizing couplings between a first and a second component of a system, wherein one of the two components contains an item of information relating to this first component, wherein a checking unit recognizes and processes this item of information and exerts an effect on the second component, wherein, as a function of a result of the processing of the item of information, the checking unit allows or prevents coupling between the first and the second component, **characterized in that** the checking unit opens a barrier in the case of an admissible coupling, wherein the barrier prevents a connection between the first and second coupling elements in the case of an inadmissible coupling.

## Revendications

1. Arrangement pour reconnaître des couplages entre un premier et un deuxième composant d'un système, l'arrangement possédant les deux composants, une unité d'identification et une unité de contrôle, l'un des deux composants étant pourvu de l'unité d'identification et l'unité de contrôle étant en liaison fonctionnelle avec le deuxième composant, l'unité d'identification contenant une information à propos du premier composant et l'unité de contrôle étant configurée pour reconnaître et traiter cette information, l'unité de contrôle étant en outre configurée de telle sorte qu'elle autorise ou empêche un couplage entre le premier et le deuxième composant en fonction d'un résultat du traitement de l'information, **caractérisé en ce que** l'unité de contrôle est en liaison fonctionnelle avec une barrière et **en ce que** l'unité de contrôle autorise ou empêche une liaison entre le premier et le deuxième composant au moyen de la barrière, la barrière empêchant une liaison entre le premier et le deuxième élément de couplage dans le cas d'un couplage non autorisé.

2. Arrangement selon la revendication 1, le deuxième composant étant un tire-lait destiné au pompage du lait maternel humain ou une pompe de drainage destinée au pompage de liquides corporels.

3. Arrangement selon l'une des revendications 1 ou 2, le premier composant étant un accessoire du deuxième composant, de préférence une téterelle d'un tire-lait destiné au pompage du lait maternel humain ou un tuyau d'aspiration ou un tuyau de service ou un insert de blessure ou un récipient collecteur de fluide ou un cathéter.

4. Arrangement selon l'une des revendications 1 à 3, le premier composant possédant un premier élément de couplage et le deuxième composant un deuxième élément de couplage et les deux éléments de couplage étant reliés l'un à l'autre en vue de créer le couplage.

5. Arrangement selon la revendication 4, le premier et le deuxième élément de couplage étant deux parties mutuellement complémentaires d'une liaison par enfichage ou d'une liaison par encliquetage ou d'une liaison par rotation ou d'une liaison par baïonnette ou d'une liaison par aimant ou d'une liaison électromécanique.

6. Arrangement selon la revendication 4, l'unité d'identification et l'unité de contrôle étant configurées de telle sorte qu'une reconnaissance et un traitement de l'information s'effectuent lorsque le couplage des premier et deuxième éléments de couplage n'est pas effectué.

7. Arrangement selon l'une des revendications 1 à 6, l'unité de contrôle possédant un dispositif d'identification par radiofréquence (RFID).

8. Arrangement selon l'une des revendications 1 à 7, l'unité d'identification et l'unité de contrôle étant configurées de telle sorte que la communication et/ou la reconnaissance et/ou le traitement de l'information sont effectués de manière électronique ou optique ou chimique.

9. Arrangement selon l'une des revendications 1 à 8, l'unité d'identification et l'unité de contrôle étant configurées de telle sorte que la communication de l'information s'effectue sans contact.

10. Arrangement selon l'une des revendications 1 à 9, l'unité de reconnaissance étant en liaison fonctionnelle avec un commutateur qui, lorsque le couplage est autorisé, libère le fonctionnement de la pompe d'aspiration.

11. Arrangement selon la revendication 1, la barrière étant disposée sur ou dans la deuxième partie de couplage.

12. Arrangement selon l'une des revendications 1 à 11, le système étant un système tire-lait destiné au pompage du lait maternel humain ou un système de drainage destiné au pompage de liquides corporels.

13. Arrangement selon l'une des revendications 1 à 12, l'unité de contrôle étant en outre configurée de telle sorte qu'elle autorise ou empêche un fonctionnement du deuxième composant, qui est dans ce cas une pompe d'aspiration, en fonction du résultat du traitement de l'information.

14. Procédé pour reconnaître des couplages entre un premier et un deuxième composant d'un système, l'un des deux composants contenant une information à propos de ce premier composant, une unité de contrôle reconnaissant et traitant cette information et exerçant un effet sur le deuxième composant, l'unité de contrôle autorisant ou empêchant un couplage entre le premier et le deuxième composant en fonction d'un résultat du traitement de l'information, **caractérisé en ce que** l'unité de contrôle, lorsqu'un couplage est autorisé, ouvre une barrière qui empêche une liaison entre le premier et le deuxième élément de couplage dans le cas d'un couplage non autorisé.
